(19) Europäisches Patentamt
European Patent Office
Office européen des brevets

(11) EP 1 312 614 A1

(12) EUROPEAN PATENT APPLICATION

(43) Date of publication:
**21.05.2003 Bulletin 2003/21**

(51) Int Cl.[7]: **C07K 14/52**, C12Q 1/68

(21) Application number: **01402950.8**

(22) Date of filing: **16.11.2001**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **GenOdyssee**
**91974 Courtaboeuf (FR)**

(72) Inventor: **Escary, Jean-Louis**
**78150 Le Chesnay (FR)**

(74) Representative: **Santarelli**
**14, avenue de la Grande Armée,**
**B.P. 237**
**75822 Paris Cedex 17 (FR)**

(54) **Polynucleotides and polypeptides of the GCP-2 gene**

(57) The present invention relates to new polynucleotides deriving from the nucleotide sequence of the GCP-2 gene and comprising new SNPs, new polypeptides derived from the natural protein GCP-2 and comprising a mutation caused by the SNPs of the invention as well as their therapeutic uses.



Printed by Jouve, 75001 PARIS (FR)

LISTE DE SEQUENCES

<110> Genodyssee

<120> New polynucleotides and polypeptides of GCP-2 gene

<130> GCP-2

<140>
<141>

<160> 9

<170> PatentIn Ver. 2.1

<210> 1
<211> 3460
<212> ADN
<213> Homo sapiens

<400> 1
aagaaagtca ttggtagctt gatggggatg gtattgaatc tataagttac cttgggcagt 60
atggccatat tcacgatatt gatttttcct acccatgagc atggaatatt cttccatttg 120
tttgtatcct cttttatttc attgagcagt ggtttgtagt tctccttgaa gaggtccttc 180
atgtcccttg taagttggat tcctaggtat tttattctct ttgaagcaat tgtgaatggg 240
agttcactca tgatttggct ctctgtttgt ttgttattgg tgtataagaa tgcttgtgat 300
ttttgtgcat tgattttgta tcctgagact ttgctgaagt tgcttatcag cttaaggaga 360
ttttgggctg agaccatggg gttttctaga tatacaatta tgtaatttgc aaatagggac 420
aatttgactt cctcttttcc taattgaata ccctttattt ccttctcctg cctaattgtc 480
ctggccattg gagaggagga gcatctccca gacagctgcg tgcctcagag aagccagcct 540
cgctaacccc tcaagcccag gggatgagac cctcctgaat cgctgctcta ttttggctgg 600
agccacagct ccctccaccg cggggcgggg ctaaaatgtc ctcccccctta agggagcagg 660
cagctcctcc cagccaccca ccccaccaat tcccatcctc ccgccccccct ccaccaaccc 720
cttctttcca cactgccccc tgagttcagg gaatttcccc agcatcccaa agcttgagtt 780
tcctgccagt cgggagggat gaatgcagat aaagggagtg cagaaggcac gaggaaacca 840
aagtgctctg tatcctccag tctccgcgcc tccacccagc tcaggaaccc gcgaaccctc 900
tcttgaccac tatgagcctc ccgtccagcc gcgcggcccg tgtcccgggt ccttcgggct 960
ccttgtgcgc gctgctcgcg ctgctgctcc tgctgacgcc gccggggccc ctcgccagcg 1020
gtgagagctc ctggcactgg ggtgcatccc agcctctgcg gggccgctgc gttccaggga 1080
actctcccag caacctgccc tataaaaatg tctttcttcc ccagctggtc ctgtctctgc 1140
tgtgctgaca gagctgcgtt gcacttgttt acgcgttacg ctgagagtaa accccaaaac 1200
gattggtaaa ctgcaggtgt tccccgcagg cccgcagtgc tccaaggtgg aagtggtgta 1260
agttctcctg tgttgctgtg tccactgtga cttaggcaag tcctccagcc tgggtcgtca 1320
acctttgtgg ctcatgggtg catcctcttt ttctttactt cagagcctcc ctgaagaacg 1380
ggaagcaagt ttgtctggac ccggaagccc cttttctaaa gaaagtcatc cagaaaattt 1440
tggacaggta tttgtccctt tgatctttgt ggtgttttaa tatcttctat ggaaagcata 1500
tacttcacaa tgtccttatt ctctctgtag gatttagact atgcttagaa ttataaggtt 1560
gttaagaaga ataaggaaac tttttttctg gaatgttctg ggtaaacctt tatcaccaat 1620
cttacatgcc tgaacaatta cacagagctc attactgaca tctatttttt gtctgctctt 1680
tgcttttatt gatttttttc ccccaccaaa cgcttttgaa aaccaaatgt agcatacaag 1740
agtgtgggaa ttggttatac taatataact cttttctcaa cagtggaaac aagaaaaact 1800
gagtaacaaa aaagaccatg catcataaaa ttgcccagtc ttcagcggag cagttttctg 1860
gagatccctg gacccagtaa gaataagaag gaagggttgg tttttttcca ttttctacat 1920
ggattcccta ctttgaagag tgtgggggaa agcctacgct tctccctgaa gtttacagct 1980
cagctaatga agtactaata tagtatttcc actatttact gttattttac ctgataagtt 2040
attgaaccct ttggcaattg accatattgt gagcaaagaa tcactggtta ttagtctttc 2100
aatgaatatt gaattgaaga taactattgt atttctatca tacattcctt aaagtcttac 2160

cgaaaaggct gtggatttcg tatggaaata atgttttatt agtgtgctgt tgagggaggt 2220
atcctgttgt tcttactcac tcttctcata aaataggaaa tattttagtt ctgtttcttg 2280
gggaatatgt tactctttac cctaggatgc tatttaagtt gtactgtatt agaacactgg 2340
gtgtgtcata ccgttatctg tgcagaatat atttccttat tcagaatttc taaaaattta 2400
agttctgtaa gggctaatat attctcttcc tatggtttta gacgtttgat gtcttcttag 2460
tatggcataa tgtcatgatt tactcattaa actttgattt tgtatgctat tttttcacta 2520
taggatgact ataattctgg tcactaaata tacactttag atagatgaag aagcccaaaa 2580
acagataaat tcctgattgc taatttacat agaaatgtat tctcttggtt ttttaaataa 2640
aagcaaaatt aacaatgatc tgtgctctga aagttttgaa aatatatttg aacaatttga 2700
atataaattc atcatttagt cctcaaaata tatacagcat tgctaagatt ttcagatatc 2760
tattgtggat cttttaaagg ttttgaccat tttgttatga ggaattatac atgtatcaca 2820
ttcactatat taaaattgca cttttatttt ttcctgtgtg tcatgttggt ttttggtact 2880
tgtattgtca tttggagaaa caataaaaga tttctaaacc actgatgttg tttctccttc 2940
ttatacagtt actatttatc tttaattcta cattattcaa aatattacct ctgctcttct 3000
ctggctggca gagaggccct cattacccaa taccattgca ttggttcaac ttttctccat 3060
gttcagcccc cttccagtta ctccttcaca gcaccaatag cctctggggt ctttagaaaa 3120
cacaaatagg ataagatttt cctatctaaa ttcttaaatg gctccctgtt tcctagacat 3180
gaaataaaag ttgctaaaca tgatgaatga ggttctgtct catctcactc ctgatcatcg 3240
gtacttcaac ttcccttgtg cctcacattc actatagtca ggcgttcagt tccctaacta 3300
ggcatgttct ttccccaggc tcatgacttt gtatttgcta gggtctctac ctggaaagca 3360
tttacgtttt cctgcgtata agaggaggct tattcatcct tcagaactca gttcaagcaa 3420
tatctccttc gtgaattttc cttggcacac tcagcaaagc 3460

<210> 2
<211> 114
<212> PRT
<213> Homo sapiens

<400> 2
Met Ser Leu Pro Ser Ser Arg Ala Ala Arg Val Pro Gly Pro Ser Gly
1 5 10 15

Ser Leu Cys Ala Leu Leu Ala Leu Leu Leu Leu Leu Thr Pro Pro Gly
20 25 30

Pro Leu Ala Ser Ala Gly Pro Val Ser Ala Val Leu Thr Glu Leu Arg
35 40 45

Cys Thr Cys Leu Arg Val Thr Leu Arg Val Asn Pro Lys Thr Ile Gly
50 55 60

Lys Leu Gln Val Phe Pro Ala Gly Pro Gln Cys Ser Lys Val Glu Val
65 70 75 80

Val Ala Ser Leu Lys Asn Gly Lys Gln Val Cys Leu Asp Pro Glu Ala
85 90 95

Pro Phe Leu Lys Lys Val Ile Gln Lys Ile Leu Asp Ser Gly Asn Lys
100 105 110

Lys Asn

<210> 3
<211> 115

<212> PRT
<213> Homo sapiens

<400> 3
Met Ser Leu Pro Ser Ser Arg Ala Ala Arg Val Pro Gly Pro Ser Gly
1               5                   10                  15

Ser Leu Cys Ala Leu Leu Ala Leu Leu Leu Leu Leu Thr Pro Pro Gly
            20                  25                  30

Pro Leu Ala Ser Ala Gly Pro Val Ser Ala Val Leu Thr Glu Leu Arg
        35                  40                  45

Cys Thr Cys Leu Arg Val Thr Leu Arg Val Asn Pro Lys Thr Ile Gly
    50                  55                  60

Lys Leu Gln Val Phe Pro Ala Gly Pro Gln Cys Ser Lys Val Glu Val
65                  70                  75                  80

Gly Ser Leu Pro Glu Glu Arg Glu Ala Ser Leu Ser Gly Pro Gly Ser
                85                  90                  95

Pro Phe Ser Lys Glu Ser His Pro Glu Asn Phe Gly Gln Trp Lys Gln
            100                 105                 110

Glu Lys Leu
        115

<210> 4
<211> 19
<212> ADN
<213> Homo sapiens

<400> 4
cagcaacctg ccctataaa 19

<210> 5
<211> 20
<212> ADN
<213> Homo sapiens

<400> 5
acttgcctaa gtcacagtgg 20

<210> 6
<211> 19
<212> ADN
<213> Homo sapiens

<400> 6
attggagagg aggagcatc 19

<210> 7

```
<211> 16
<212> ADN
<213> Homo sapiens

<400> 7
aaggagcccg aaggac                                                  16


<210> 8
<211> 21
<212> ADN
<213> Homo sapiens

<400> 8
gtgctctgta tcctccagtc t                                            21


<210> 9
<211> 19
<212> ADN
<213> Homo sapiens

<400> 9
tttatagggc aggttgctg                                               19
```

## Description

The present invention relates to new polynucleotides deriving from the nucleotide sequence of the GCP-2 gene and comprising new SNPs, new polypeptides derived from the natural protein GCP-2 and comprising mutations caused by these SNPs as well as their therapeutic uses.

PRIOR ART

GCP-2 (Ganulocyte Chemotactic Peptide-2) is a factor of 6 kDa (75 amino acids) which is produced by stimulated human osteosarcoma cells (MG-63).

It is co-produced in minute amounts together with IL8. GCP-2 is structurally related to the other members of the IL8 family and belongs to the family of chemotactic cytokines known as Chemokines (C-X-C Chemokines). A characteristic feature of this factor is the presence of a conserved ELR sequence motif.

The protein is known also as SCY B6 (small inductible cytokine subfamily B member 6) and the sequence is identical to that of CKA-3.

Four different forms of human GCP-2, showing differences in truncation at the amino terminus, have been described. The first form corresponds to the mature protein lacking the signal sequence, the second one additionally lacks the 38 and 39 amino acids, the third one additionally lacks the 38 to 42 amino acids, and the fourth one additionally lacks the 38 to 45 amino acids.

Human and bovine GCP-2 are 67 percent identical at the amino acid level. Murine GCP-2 (isolated from mouse fibroblasts and epithelial cells) and human or bovine GCP-2 have 61 and 64 percent identical residues, respectively. Amino-terminal cleavage of murine GCP-2 generates 11 different forms of the protein. GCP-2 sequences show only weak similarity with that of IL8, and human GCP-2 does not crossreact in a radioimmunoassay for IL8.

In diploid fibroblasts and epithelial cells, expression of GCP-2 is induced by IL1 and downregulated by IFN-gamma. GCP-2 expression is induced in MG-63 cells (but not A549 cells) by TNF-alpha, IL1-beta, and bacteria! lipopolysaccharides.

The chemoattractive potency of GCP-2 for neutrophilic granulocytes at optimal dose (100 ng/ml) is comparable with that of IL8. GCP-2 provokes a significant granulocyte infiltration in a rabbit skin model involving intradermal injection, albeit to a lesser extent than IL8 and related factors. GCP-2 does not attract monocytes in vivo.

Short forms of murine GCP-2 have a higher specific activity in neutrophil activation (gelatinase B release) and chemotaxis assays. Murine GCP-2 is more potent than human GCP-2 on human neutrophils, and more active than murine KC and MIP-2 on mouse neutrophils.

GCP-2 appears to utilize the receptors for IL8, CXCR-1 and CXCR-2.

GCP-2 is mentioned in the following references :

- Ahuja SK and Murphy PM; The CXC chemokines growth-regulated oncogene (GRO) alpha, GRObeta, GROgamma, neutrophil-activating peptide-2, and epithelial cell-derived neutrophil-activating peptide-78 are potent agonists for the type B, but not the type A, human interleukin-8 receptor. Journal of Biological Chemistry 271(34): 20545-20550 (1996).
- Froyen G et al.; Cloning, bacterial expression and biological characterization of recombinant human granulocyte chemotactic protein 2 and differential expression of granulocyte chemotactic protein 2 and epithelial cell derived neutrophil activating peptide 78 mRNAs. European Journal of Biochemistry 243(3): 762-769 (1997).
- Proost P et al.; Human and bovine granulocyte chemotactic protein-2: complete amino acid sequence and functional characterization as chemokines. Biochemistry 32: 10170-10177 (1993).
- Proost P et al.; Identification of a novel granulocyte chemotactic protein (GCP-2) from human tumor cells. In vitro and in vivo comparison with natural forms of GRO, IP-10, and IL8. Journal of Immunology 150: 1000-10 (1993).
- Rovai LE et al Cloning and characterization of the human granulocyte chemotactic protein-2 gene. Journal of Immunology 158(11): 5257-66 (1997).
- Wuyts A et al.; Identification of mouse granulocyte chemotactic protein 2 from fibroblasts and epithelial cells: functional comparison with natural kc and macrophage inflammatory protein 2. Journal of Immunology 157(4): 1736-1743 (1996).
- Wuyts A et al.; Characterization of synthetic human granulocyte chemotactic protein 2: usage of chemokine receptors CXCR1 and CXCR2 and in vivo inflammatory properties. Biochemistry 36(9): 2716-23 (1997).

IL8, also called GCP (granulocyte chemotactic peptide), is produced by stimulated monocytes but not by tissue macrophages and T-lymphocytes. IL8 is produced also by macrophages, fibroblasts, endothelial cells, keratinocytes, melanocytes, hepatocytes, chondrocytes, and a number of tumor cell lines.

In many cell types the synthesis of IL8 is strongly stimulated by IL1 and TNF alpha. In human skin fibroblasts the expression of IL8 is enhanced by leukoregulin. The interferon IFN-gamma can function as a costimulator. The synthesis of IL8 is induced also by phytohemagglutinins, concanavalin A, double-stranded RNA, Phorbol ester, sodium urate crystals, viruses, and bacterial lipopolysaccharides. The expression of IL8 from resting and stimulated human blood monocytes is up-regulated by IL7. In chondrocytes the synthesis of IL8 is stimulated by IL1-beta, TNF-alpha and lipopolysaccharides. In human astrocytes the synthesis and secretion of IL8 is induced by IL1 and TNF-alpha. Glucocorticoids, IL4, TGF-beta, inhibitors of 5' lipoxygenase, and 1,25$(OH)_2$ vitamin D3 inhibit the synthesis of IL8. IL8 is constitutively and commonly produced by various carcinoma cell lines and this synthesis may be related to the elevation of serum IL8 in patients with hepatocellular carcinoma. In epithelial, endothelial, and fibroblastic cells secretion of IL8 is induced by IL17.

IL8 may be of clinical relevance in psoriasis and rheumatoid arthritis. Elevated concentrations are observed in psoriatic scales and this may explain the high proliferation rate observed in these cells. IL8 may be also a marker of different inflammatory processes.

IL8 probably plays a role in the pathogenesis of chronic polyarthritis since excessive amounts of this factor are found in synovial fluids. The activation of neutrophils may enhance the migration of cells into the capillaries of the joints. These cells are thought to pass through the capillaries and enter the surrounding tissues, thus causing a constant stream of inflammatory cells through the joints.

Using Human recombinant IL8, it has been shown that the lesion responsible for defective neutrophil functions in patients with myelodysplastic syndrome can be restored without stimulating myeloid progenitor cells. Thus, IL8 may be able to reduce the risks of lethal infections in these patients without the potential risk of stimulating leukemic clones.

It appears that the GCP-2 gene may be involved in different human disorders and/or diseases involving the immune system, inflammation and tumor cell invasion, psoriasis and rheumatoid arthritis, chronic polyarthritis, myelodysplastic syndrome, carcinoma such as hepatocellular carcinoma and osteosarcoma.

The inventors have found new polypeptides and new polynucleotides analogs to the GCP-2 gene capable of having a different functionality from the natural GCP-2 protein.

## THE INVENTION

The invention has as its first object new polynucleotides that differ from the nucleotide sequence of the reference wild-type GCP-2 gene, in that it comprises one or several SNPs (Single Nucleotide Polymorphism).

The nucleotide sequence SEQ ID N°1 of the human reference wild-type GCP-2 gene is composed of 3460 nucleotides and comprises the four following exons:

- Exon 1 : nucleotide 912 to nucleotide 1020;
- Exon 2 : nucleotide 1125 to nucleotide 1257;
- Exon 3 : nucleotide 1364 to nucleotide 1447; and
- Exon 4 : nucleotide 1784 to nucleotide 1802;

The nucleotide sequence SEQ ID N°1 comprises a coding sequence of 345 nucleotides, with the four exons, from nucleotide 912 (start codon) to the nucleotide 1802 (stop codon).

The applicant has identified seven SNPs in the nucleotide sequence of the reference wild-type GCP-2 gene.

These SNPs are the following: g563t, c574g, c646g, g742t, a1026t, g1069c and 1254-1255 Ins (t).

It is understood, in the sense of the present invention, that the numbering corresponding to the positioning of the SNP previously defined is relative to the numbering of the nucleotide sequence SEQ ID N°1. If a specific portion of the nucleotide sequence SEQ ID N°1 is isolated, it is understood that the SNP which is comprised in this portion is renumbered accordingly. For example, if a portion of the nucleotide sequence SEQ ID N°1 comprising nucleotides 500 to 600 is isolated, then the SNP g563t becomes g63t on said portion of the sequence.

The letters a, t, c and g correspond respectively to the nitrogenous bases adenine, thymine, cytosine and guanine.

The first letter corresponds to the wild-type nucleotide, whereas the last letter corresponds to the mutated nucleotide.

Thus, for example, the SNP g563t corresponds to a mutation of the nucleotide guanine (g) at position 563 of the nucleotide sequence SEQ ID N°1 of the reference wild-type GCP-2 gene, to a thymine (t).

The SNP 1254-1255 Ins (t) corresponds to the insertion of a thymine (t) between the nucleotide 1254 and 1255.

These SNPs have each been identified by the applicant using the determination process described in applicant's patent application FR 00 22894, entitled "Process for the determination of one or several functional polymorphism (s) in the nucleotide sequence of a preselected functional candidate gene and its applications" (Procédé de détermination d'un ou plusieurs polymorphisme(s) fonctionnel(s) dans la séquence nucléique d'un gène "candidat" fonctionnel présélectionné et ses applications) and filed December 6, 2000, cited here by way of reference.

The process described in this patent application permits the identification of one (or several) preexisting SNP (s) in at least one individual from a random population of individuals.

In the scope of the present invention, a fragment of the nucleotide sequence of the GCP-2 gene, comprising, for example, the coding sequence, was isolated from different individuals in a population of individuals chosen in a random manner.

Sequencing of these fragments was then carried out on certain of these samples having a heteroduplex profile (that is a profile different from that of the reference wild-type GCP-2 gene sequence) after analysis by DHPLC ("Denaturing-High Performance Liquid Chromatography").

The fragment sequenced in this way was then compared to the nucleotide sequence of the fragment of the reference wild-type GCP-2 gene and the SNPs in conformity with the invention identified.

Thus, the SNPs are natural and each of them is present in certain individuals of the world population.

The reference wild-type GCP-2 gene encodes for an immature protein of 114 amino acids, corresponding to the amino acid sequence SEQ ID N°2, that will be converted to a mature protein of 77 amino acids, by cleavage of the signal peptide that includes the 37 first amino acids.

The coding SNP of the invention : 1254-1255 Ins (t) causes modifications of the protein encoded by the nucleotide sequence of the GCP-2 gene at the level of the amino acid sequence.

The SNP 1254-1255 Ins (t) causes an insertion of a thymine (t) after position 1254 of the nucleotide sequence SEQ ID N°1 of the GCP-2 gene, in exon 2. This insertion creates a frameshift in said sequence. The resulting protein, encoded by the SEQ ID N°1 comprising the SNP 1254-1255 Ins (t), has the same sequence as the wild-type protein for the first 80 amino acids. The rest of the resulting protein is completely different after the amino acid number 80.

The immature mutated amino acid sequence corresponds to the amino acid sequence SEQ ID N°3. This amino acid sequence comprises 115 amino acids. The mature mutated amino acid sequence comprises 78 amino acids obtained by cleavage of the signal peptide that includes the 37 first amino acids.

This abnormal mutated protein is certainly unable to functionally replace the wild-type protein.

Other SNPs in conformity with the invention, namely: g563t, c574g, c646g, g742t, a1026t and g1069c do not involve modification of the protein encoded by the nucleotide sequence of the GCP-2 gene at the level of the amino acid sequence SEQ ID N°2. These SNPs are non-coding.

Genotyping of the polynucleotides in conformity with the invention can be carried out in such a fashion as to determine the allelic frequency of these polynucleotides in a population. An example of genotyping is given, hereinafter, in the experimental part.

The invention also has for an object the use of polynucleotides and of polypeptides in conformity with the invention as well as of therapeutic molecules obtained and/or identified starting from these polynucleotides and polypeptides, notably for the prevention and the treatment of certain human disorders and/or diseases.

Figure 1 represents the result of the genotyping of the SNP 1254-1255 Ins (t) in a population of individuals.

On this figure, the abscissas represent the mP value of the Tamra filter (ddTTP) and the ordinates represent the mP value of the R-110 filter (ddGTP).

At the upper right, the heterozygote group TG contains 3 individuals.

At the upper left, the homozygote group GG contains 263 individuals.

At the lower left, the group of individuals contains 8 negative-controls and 2 non-genotyped individuals.

## DETAILED DESCRIPTION OF THE INVENTION

### Definitions

By "nucleotide sequence of the reference wild-type gene" is understood the nucleotide sequence SEQ ID N° 1 of the human gene.

This sequence is accessible in GenBank under Accession number U83303 and described in Rovai et al., (1997); "Cloning and characterization of the human granulocyte chemotactic protein-2 gene"; J. Immunol.; 158, 5257-5266.

By "natural GCP-2 protein" is understood the mature protein encoded by the nucleotide sequence of the reference wild-type GCP-2 gene. The natural immature protein GCP-2 corresponds to the peptide sequence SEQ ID N°2.

By "polynucleotide" is understood a polyribonucleotide or a polydeoxyribonucleotide that can be a modified or non-modified DNA or an RNA.

The term polynucleotide includes, for example, a single strand or double strand DNA, a DNA composed of a mixture of one or several single strand region(s) and of one or several double strand region(s), a single strand or double strand RNA and an RNA composed of a mixture of one or several single strand region(s) and of one or several double strand region(s). The term polynucleotide can also include an RNA and/or a DNA including one or several triple strand regions. By polynucleotide is equally understood the DNAs and RNAs containing one or several bases modified in such a fashion as to have a skeleton modified for reasons of stability or for other reasons. By modified base is understood, for example, the unusual bases such as inosine.

By "polypeptide" is understood a peptide, an oligopeptide, an oligomer or a protein comprising at least two amino acids joined to each other by a normal or modified peptide bond, such as in the cases of the isosteric peptides, for example.

A polypeptide can be composed of amino acids other than the 20 amino acids defined by the genetic code. A polypeptide can equally be composed of amino acids modified by natural processes, such as post translational maturation processes or by chemical processes, which are well known to a person skilled in the art. Such modifications are fully detailed in the literature. These modifications can appear anywhere in the polypeptide: in the peptide skeleton, in the amino acid chain or even at the carboxy- or amino-terminal ends.

A polypeptide can be branched following an ubiquitination or be cyclic with or without branching. This type of modification can be the result of natural or synthetic post-translational processes that are well known to a person skilled in the art.

For example, by polypeptide modifications is understood acetylation, acylation, ADP-ribosylation, amidation, covalent fixation of flavine, covalent fixation of heme, covalent fixation of a nucleotide or of a nucleotide derivative, covalent fixation of a lipid or of a lipidic derivative, the covalent fixation of a phosphatidylinositol, covalent or non-covalent cross-linking, cyclization, disulfide bridge formation, demethylation, cysteine formation, pyroglutamate formation, formylation, gamma-carboxylation, glycosylation, GPI anchor formation, hydroxylation, iodization, methylation, myristoylation, oxidation, proteolytic processes, phosphorylation, prenylation, racemization, seneloylation, sulfatation, amino acid addition such as arginylation or ubiquitination. Such modifications are fully detailed in the literature: PROTEINS-STRUCTURE AND MOLECULAR PROPERTIES, 2$^{nd}$ Ed., T. E. Creighton, New York, 1993, POST-TRANSLATIONAL COVALENT MODIFICATION OF PROTEINS, B. C. Johnson, Ed., Academic Press, New York, 1983, Seifter et al. "Analysis for protein modifications and nonprotein cofactors", Meth. Enzymol. (1990) 182: 626-646 et Rattan et al. "Protein Synthesis: Post-translational Modifications and Aging", Ann NY Acad Sci (1992) 663: 48-62.

By "isolated polynucleotide" or "isolated polypeptide" is understood a polynucleotide or a polypeptide such as previously defined which is isolated from the human body or otherwise produced by a technical process.

By "identity" is understood the measurement of nucleotide or polypeptide sequence identity.

Identity is a term well known to a person skilled in the art and well described in the literature. See COMPUTATIONAL MOLECULAR BIOLOGY, Lesk, A.M., Ed., Oxford University Press, New York, 1998; BIOCOMPUTING INFORMATICS AND GENOME PROJECT, Smith, D.W., Ed., Academic Press, New York, 1993; COMPUTER ANALYSIS OF SEQUENCE DATA, PART I, Griffin, A.M. and Griffin H.G., Ed, Humana Press, New Jersey, 1994; et SEQUENCE ANALYSIS IN MOLECULAR BIOLOGY, von Heinje, G., Academic Press, 1987.

The methods commonly employed to determine the identity and the similarity between two sequences are equally well described in the literature. See GUIDE TO HUGE COMPUTER, Martin J. Bishop, Ed, Academic Press, San Diego, 1994, et Carillo H. and Lipton D., Siam J Applied Math (1988) 48:1073.

A polynucleotide having, for example, an identity of at least 95 % with the nucleotide sequence SEQ ID N° 1 is a polynucleotide which contains at most 5 points of mutation over 100 nucleotides, compared to said sequence.

These points of mutation can be one (or several) substitution(s), addition(s) and/or deletion(s) of one (or several) nucleotide(s).

In the same way, a polypeptide having, for example, an identity of at least 95 % with the amino acid sequence SEQ ID N° 3 is a polypeptide that contains at most 5 points of mutation over 100 amino acids, compared to said sequence.

These points of mutation can be one (or several) substitution(s), addition(s) and/or deletion(s) of one (or several) amino acid(s).

The polynucleotides according to the invention which are not totally identical with the nucleotide sequence SEQ ID N°1, it being understood that this sequence contains at least one of the SNPs of the invention, are considered as variants of these sequences.

The polypeptides according to the invention which are not totally identical with the amino acid sequence SEQ ID N°3 are considered as variants of this sequence.

A variant, according to the invention, can be obtained, for example, by site-directed mutagenesis or by direct synthesis.

By "SNP" is understood any natural variation of a base in a nucleotide sequence. A SNP, on a nucleotide sequence, can be coding, silent or non-coding.

A coding SNP is a polymorphism included in the coding sequence of a nucleotide sequence that involves a modification of at least one amino acid in the sequence of amino acids encoded by this nucleotide sequence. In this case, the term SNP applies equally, by extension, to a mutation in an amino acid sequence.

A silent SNP is a polymorphism included in the coding sequence of a nucleotide sequence that does not involve a modification of an amino acid in the amino acid sequence encoded by this nucleotide sequence.

A non-coding SNP is a polymorphism included in the non-coding sequence of a nucleotide sequence. This polymorphism can notably be found in an intron, a splicing zone, a transcription promoter or a site enhancer sequence.

By "functional SNP" is understood a SNP, such as previously defined, which is included in a nucleotide sequence or an amino acid sequence, having a functionality.

By "functionality" is understood the biological activity of a polypeptide or of a polynucleotide.

The functionality of a polypeptide or of a polynucleotide according to the invention can consist in a conservation, an augmentation, a reduction or a suppression of the biological activity of the polypeptide encoded by the nucleotide sequence of the wild reference gene or of this latter nucleotide sequence.

The functionality of a polypeptide or of a polynucleotide according to the invention can equally consist in a change in the nature of the biological activity of the polypeptide encoded by the nucleotide sequence of the reference wild-type gene or of this latter nucleotide sequence.

The biological activity can, notably, be linked to the affinity or to the absence of affinity of a polypeptide according to the invention with a receptor.

Polynucleotide

The present invention has for its first object an isolated polynucieotide comprising:

a) a nucleotide sequence having at least 80 % identity, preferably at least 90 % identity, more preferably at least 95 % identity and still more preferably at least 99 % identity with the sequence SEQ ID N° 1 or its coding sequence (of the nucleotide 912 to the nucleotide 1802), it being understood that this nucleotide sequence comprises the following coding SNP 1254-1255 Ins (t); or

b) a nucleotide sequence complementary to a nucleotide sequence under a).

The present invention relates equally to an isolated polynucleotide comprising:

a) the nucleotide sequence SEQ ID N° 1 or its coding sequence, it being understood that each of these sequences comprises the following coding SNP: 1254-1255 Ins (t), or

b) a nucleotide sequence complementary to a nucleotide sequence under a).

Preferably, the polynucleotide of the invention consists of the sequence SEQ ID N° 1 or its coding sequence, it being understood that each of these sequences comprises the following coding SNP : 1254-1255 Ins (t).

A polynucleotide such as previously defined can equally include at least one of the following non-coding SNPs : g563t, c574g, c646g, g742t, a1026t and g1069c.

The present invention equally has for its object an isolated polynucleotide comprising or consisting of:

a) a nucleotide sequence having at least 80 % identity (preferably at least 90 % identity, more preferably at least 95 % identity and still more preferably at least 99 % identity or even 100% identity) with the sequence SEQ ID N° 1, it being understood that this sequence comprises at least one of the following non coding SNPs : g563t, c574g, c646g, g742t, a1026t, g1069c, or

b) a nucleotide sequence complementary to the nucleotide sequence under a).

The present invention concerns also an isolated polynucleotide consisting of a part of a polynucleotide according to the invention, such as previously defined, it being understood that the sequence of said isolated polynucleotide contains at least one of the following SNP g563t, c574g, c646g, g742t, a1026t, g1069c and 1254-1255 Ins (t), said isolated nucleotide is composed of at least 10 nucleotides.

Preferably, the isolated polynucleotide as defined above is composed of 10 to 40 nucleotides.

The present invention also has for its object an isolated polynucleotide coding for a polypeptide comprising:

a) the amino acid sequence SEQ ID N° 3,

b) the amino acid sequence comprising the amino acids included between positions 38 and 115 of the sequence of amino acids SEQ ID N° 3, or

c) the amino acid sequence comprising the amino acids included between positions 46 and 115 of the amino acid sequence SEQ ID N° 3.

Preferably a polynucleotide according to the invention is composed of a DNA or RNA molecule.

A polynucleotide according to the invention can be obtained by standard DNA or RNA synthetic methods.

A polynucleotide according to the invention can equally be obtained by site-directed mutagenesis starting from the nucleotide sequence of the GCP-2 gene by modifying the wild-type nucleotide by the mutated nucleotide for each SNP on the nucleotide sequence SEQ ID N° 1.

The processes of site-directed mutagenesis that can be implemented in this way are well known to a person skilled in the art. The publication of TA Kunkel in 1985 in "Proc. Natl. Acad. Sci. USA" 82:488 can notably be mentioned.

An isolated polynucleotide can equally include, for example, nucleotide sequences coding for pre-, pro- or pre-pro-protein amino acid sequences or marker amino acid sequences, such as hexa-histidine peptide.

A polynucleotide of the invention can equally be associated with nucleotide sequences coding for other proteins or protein fragments in order to obtain fusion proteins or other purification products.

A polynucleotide according to the invention can equally include nucleotide sequences such as the 5' and/or 3' non-coding sequences, such as, for example, transcribed or non-transcribed sequences, translated or non-translated sequences, splicing signal sequences, polyadenylated sequences, ribosome binding sequences or even sequences which stabilize mRNA.

A nucleotide sequence complementary to the nucleotide or polynucleotide sequence is defined as one that can be hybridized with this nucleotide sequence, under stringent conditions.

By "stringent hybridization conditions" is generally but not necessarily understood the chemical conditions that permit a hybridization when the nucleotide sequences have an identity of at least 80 %, preferably greater than or equal to 90 %, still more preferably greater than or equal to 95 % and most preferably greater than or equal to 97 %.

The stringent conditions can be obtained according to methods well known to a person skilled in the art and, for example, by an incubation of the polynucleotides, at 42° C, in a solution comprising 50 % formamide, 5xSSC (150 mM of NaCl, 15 mM of trisodium citrate), 50 mM of sodium phosphate (pH = 7.6), 5x Denhardt Solution, 10 % dextran sulfate and 20 μg denatured salmon sperm DNA, followed by washing the filters at 0.1x SSC, at 65° C.

Within the scope of the invention, when the stringent hybridization conditions permit hybridization of the nucleotide sequences having an identity equal to 100 %, the nucleotide sequence is considered to be strictly complementary to the nucleotide sequence such as described under a).

It is understood within the meaning of the present invention that the nucleotide sequence complementary to a nucleotide sequence comprises at least one anti-sense SNP according to the invention.

Thus, for example, if the nucleotide sequence comprises the SNP 1254-1255 Ins (t), its complementary nucleotide sequence still comprises the adenine (a) nucleotide at the same position.

## Identification, hybridization and/or amplification of a polynucleotide comprising a SNP

The present invention also has for its object the use of all or part of a previously defined polynucleotide, in order to identify, hybridize and/or amplify all or part of a polynucleotide consisting of:

- the sequence SEQ ID N° 1, it being understood that this sequence contains at least one of the following SNPs : g563t, c574g, c646g, g742t, a1026t, g1069c, 1254-1255 Ins (t), and/or
- the coding sequence of the nucleotide sequence SEQ ID N° 1, it being understood that this sequence contains the following SNP : 1254-1255 Ins (t).

It is understood that only the following SNP : 1254-1255 Ins (t) can be comprised in the coding sequence of the nucleotide sequence SEQ ID N° 1.

## Genotyping and determination of the frequency of a SNP

The present invention equally has for its object the use of all or part of a polynucleotide according to the invention for the genotyping of a nucleotide sequence which has 90 to 100 % identity with the nucleotide sequence SEQ ID N°1 and which comprises at least one of the following SNPs : g563t, c574g, c646g, g742t, a1026t, g1069c and 1254-1255 Ins (t).

According to the invention, the genotyping may be carried out on an individual or a population of individuals.

Within the meaning of the invention, genotyping is defined as a process for the determination of the genotype of an individual or of a population of individuals. Genotype consists of the alleles present at one or more specific loci.

By "population of individuals" is understood a group of determined individuals selected in random or non-random fashion. These individuals can be humans, animals, microorganisms or plants.

Usually, the group of individuals comprises at least 10 persons, preferably from 100 to 300 persons.

The individuals can be selected according to their ethnicity or according to their phenotype, notably those who are affected by the disorders and/or diseases involving the immune system, inflammation and tumor cell invasion and disorders and/or diseases such as psoriasis and rheumatoid arthritis, chronic polyarthritis, myelodysplastic syndrome, carcinoma such as hepatocellular carcinoma and osteosarcoma.

A functional SNP according to the invention is preferably genotyped in a population of individuals.

Multiple technologies exist which can be implemented in order to genotype SNPs (see notably Kwok Pharmacogenomics, 2000, vol 1, pp 95-100. "High-throughput genotyping assay approaches"). These technologies are based on one of the four following principles: allele specific oligonucleotide hybridization, oligonucleotide elongation by dideoxynucleotides optionally in the presence of deoxynucleotides, ligation of allele specific oligonucleotides or cleavage of allele specific oligonucleotides. Each one of these technologies can be coupled to a detection system such as measurement of direct or polarized fluorescence, or mass spectrometry.

Genotyping can notably be carried out by minisequencing with hot ddNTPs (2 different ddNTPs labeled by different fluorophores) and cold (2 non labeled ddNTPs), in connection with a polarized fluorescence scanner. The minisequencing protocol with reading of polarized fluorescence (Technology FP-TDI or Fluorescence Polarization Template-direct Dye-Terminator Incorporation) is well known to a person skilled in the art.

It can be carried out on a product obtained after amplification by polymerase chain reaction (PCR) of the DNA of each individual. This PCR product is selected to cover the polynucleotide genic region containing the studied SNP. After the last stage in the PCR thermocycler, the plate is then placed on a polarized fluorescence scanner for a reading of the labeled bases by using fluorophore specific excitation and emission filters. The intensity values of the labeled bases are reported on a graph.

The sense and antisense primers respectively for the PCR amplification, in the case of a SNP of the invention, can easily be selected by a person skilled in the art according to the position of the SNPs of the invention.

For example, the sense and antisense nucleotide sequences for the PCR amplification can be respectively SEQ ID N° 4 and SEQ ID N° 5, for the SNP 1254-1255 Ins (t).

These nucleotide sequences permit amplification of a fragment having a length of 214 nucleotides, of the nucleotide 1088 to the nucleotide 1301 in the nucleotide sequence SEQ ID N° 1.

For example, the sense and antisense nucleotide sequences for the PCR amplification can be respectively SEQ ID N° 6 and SEQ ID N° 7, for the SNPs g563t, c574g, c646g and g742t.

These nucleotide sequences permit amplification of a fragment having a length of 478 nucleotides, of the nucleotide 487 to the nucleotide 964 in the nucleotide sequence SEQ ID N° 1.

For example, the sense and antisense nucleotide sequences for the PCR amplification can be respectively SEQ ID N° 8 and SEQ ID N° 9, for the SNPs a1026t and g1069c.

These nucleotide sequences permit amplification of a fragment having a length of 264 nucleotides, of the nucleotide 843 to the nucleotide 1106 in the nucleotide sequence SEQ ID N° 1.

A statistical analysis of the frequency of each allele (allelic frequency) encoded by the gene comprising the SNP in the population of individuals is then achieved, which permits determination of the importance of their impact and their distribution in the different sub-groups and notably, if necessary, the diverse ethnic groups that constitute this population of individuals.

The genotyping data are analyzed in order to estimate the distribution frequency of the different alleles observed in the studied populations. The calculations of the allelic frequencies can be carried out with the help of software such as SAS-suite® (SAS) or SPLUS® (MathSoft). The comparison of the allelic distributions of a SNP of the invention across different ethnic groups of the population of individuals can be carried out by means of the software ARLEQUIN® and SAS-suite®.

Expression vector and host cell

The present invention also has for its object a recombinant vector comprising at least one polynucleotide according to the invention.

Numerous expression systems can be used, like, for example, chromosomes, episomes, derived viruses. More particularly, the recombinant vectors used can be derived from bacterial plasmids, transposons, yeast episome, insertion elements, yeast chromosome elements, viruses such as baculovirus, papilloma viruses such as SV40, vaccinia viruses, adenoviruses, fox pox viruses, pseudorabies viruses, retroviruses.

These recombinant vectors can equally be cosmid or phagemid derivatives. The nucleotide sequence can be inserted in the recombinant expression vector by methods well known to a person skilled in the art such as, for example, those that are described in MOLECULAR CLONING, A LABORATORY MANUAL (supra) Sambrook et al.

The recombinant vector can include nucleotide sequences that control the regulation of the polynucleotide expression as well as nucleotide sequences permitting the expression and the transcription of a polynucleotide of the invention and the translation of a polypeptide of the invention, these sequences being selected according to the host cells that are used.

Thus, for example, an appropriate secretion signal can be integrated in the recombinant vector so that the polypeptide, encoded by the polynucleotide of the invention, will be directed towards the opening of the endoplasmic reticulum, towards the periplasmic space, on the membrane or towards the extracellular environment.

The present invention also has for its object a host cell comprising a recombinant vector according to the invention.

The introduction of the recombinant vector in a host cell can be carried out according to methods that are well known to a person skilled in the art such as those described in BASIC METHODS IN MOLECULAR BIOLOGY, Davis et al., 1986 et MOLECULAR CLONING: A LABORATORY MANUAL, 2$^{nd}$ Ed., Cold Spring Harbor Laboratory Press, Cold Spring Harbor, N.Y., 1989, such as transfection by calcium phosphate, transfection by DEAE dextran, transfection, microinjection, transfection by cationic lipids, electroporation, transduction or infection.

The host cell can be, for example, bacterial cells such as cells of streptococci, staphylococci, *E. coli* or *Bacillus subtilis,* cells of fungi such as yeast cells and cells of *Aspergillus, Streptomyces,* insect cells such as cells of *Drosophilia* S2 and of *Spodoptera* Sf9, animal cells, such as CHO, COS, HeLa, C127, BHK, HEK 293 cells and human cells of the subject to treat or even plant cells.

The host cells can be used, for example, to express a polypeptide of the invention or as active product in pharmaceutical compositions, as will be seen hereinafter.

<u>Polypeptide</u>

The present invention also has for its object an isolated polypeptide comprising an amino acid sequence having at least 80 % identity, preferably at least 90 % identity, more preferably at least 95 % identity and still more preferably at least 99 % identity with :

a) the amino acid sequence SEQ ID N° 3,
b) the amino acid sequence comprising the amino acids included between positions 38 and 115 of the amino acid sequence SEQ ID N° 3, or
c) the amino acid sequence comprising the amino acids included between positions 46 and 115 of the amino acid sequence SEQ ID N° 3.

The amino acid sequence comprising the amino acids included between positions 38 and 115 of the amino acid sequence SEQ ID N° 3 corresponds to the mature mutated protein.

The amino acid sequence comprising the amino acids included between positions 46 and 115 of the amino acid sequence SEQ ID N° 3 corresponds to a variant of human GCP-2 showing a truncation at the amino terminus. This variant lacks the 38 to 45 amino acids.

The polypeptide of the invention can equally comprise:

a) the amino acid sequence SEQ ID N° 3,
b) the amino acid sequence containing the amino acids included between positions 38 and 115 of the amino acid sequence SEQ ID N° 3, or
c) the amino acid sequence containing the amino acids included between positions 46 and 115 of the amino acid sequence SEQ ID N° 3.

The polypeptide of the invention can more particularly consist of:

a) the amino acid sequence SEQ ID N° 3,
b) the amino acid sequence containing the amino acids included between positions 38 and 115 of the amino acid sequence SEQ ID N° 3, or
c) the amino acid sequence containing the amino acids included between positions 46 and 115 of the amino acid sequence SEQ ID N° 3.

The present invention equally has for its object a process for the preparation of the above-described polypeptide, in which a previously defined host cell is cultivated in a culture medium and said polypeptide is isolated from the culture medium.

The polypeptide can be purified starting from the host cells, according to methods well known to a person skilled in the art such as precipitation with the help of chaotropic agents such as salts, in particular ammonium sulfate, ethanol acetone or trichloroacetic acid, acid extraction; ion exchange chromatography; phosphocellulose chromatography; hydrophobic interaction chromatography; affinity chromatography; hydroxyapatite chromatography or exclusion chromatographies.

By "culture medium" is understood the medium in which the polypeptide of the invention is isolated or purified. This medium can be composed of the extracellular medium and/or the cellular lysate. Techniques well known to a person skilled in the art equally permit the latter to give back an active conformation to the polypeptide, if the conformation of said polypeptide was altered during the isolation or the purification.

## Antibodies

The present invention also has for its object a process for obtaining an immunospecific antibody.
By "antibody" is understood the monoclonal, polyclonal, chimeric, simple chain, humanized antibodies as well as the Fab fragments, including Fab or immunoglobulin expression library products.
An immunospecific antibody can be obtained by immunization of an animal with a polypeptide according to the invention.
The invention also relates to an immunospecific antibody for a polypeptide according to the invention, such as defined previously.
A polypeptide according to the invention, one of its fragments, an analog, one of its variants or a cell expressing this polypeptide can also be used to produce immunospecific antibodies.
The term "immunospecific" means that the antibody possesses a better affinity for the polypeptide of the invention than for other polypeptides known in the prior art.
The immunospecific antibodies can be obtained by administration of a polypeptide of the invention, of one of its fragments, of an analog or of an epitopic fragment or of a cell expressing this polynucleotide in a mammal, preferably non human, according to methods well known to a person skilled in the art.
For the preparation of monoclonal antibodies, typical methods for antibody production can be used, starting from cell lines, such as the hybridoma technique (Kohler et al., Nature (1975) 256: 495-497), the trioma technique, the human B cell hybridoma technique (Kozbor et al., Immunology Today (1983) 4: 72) and the EBV hybridoma technique (Cole et al., MONOCLONAL ANTIBODIES AND CANCER THERAPY, pp. 77-96, Alan R. Liss, 1985).
The techniques of single chain antibody production such as described, for example, in US Patent N° 4,946, 778 can equally be used.
Transgenic animals such as mice, for example, can equally be used to produce humanized antibodies.

## Agents interacting with the polypeptide of the invention

The present invention equally has for its object a process for the identification of an activating or inhibiting agent of a polypeptide according to the invention, comprising:

a) the preparation of a recombinant vector comprising a polynucleotide according to the invention containing the coding SNP of the invention,
b) the preparation of host cells comprising a recombinant vector according to a),
c) the contacting of host cells according to b) with an agent to be tested, and
d) the determination of the activating or inhibiting effect generated by the agent to test.

A polypeptide according to the invention can also be employed for a process for screening compounds that interact with it.
These compounds can be activating (agonists) or inhibiting (antagonists) agents of intrinsic activity of a polypeptide according to the invention. These compounds can equally be ligands or polypeptide substrates of the invention. See Coligan et al., Current Protocols in Immunology 1 (2), Chapter 5 (1991).
In general, in order to implement such a process, it is first desirable to produce appropriate host cells that express a polypeptide according to the invention. Such cells can be, for example, cells of mammals, yeasts, insects such as *Drosophilia* or bacteria such as *E. coli.*
These cells or membrane extracts of these cells are then put in the presence of compounds to be tested.
The linking capacity of the compounds to be tested with the polypeptide of the invention can then be observed, as well as the inhibition or the activation of the functional response.
Stage d) of the above process can be implemented by using a labeled agent to be tested directly or indirectly. It can also include a competition test, by using a labeled or non-labeled agent and a labeled competitor agent.
It can equally be determined if an agent to be tested leads to the generation of an activation or inhibition signal on cells expressing the polypeptide of the invention, by using appropriate detection means, following the signal to be detected.
Such activating or inhibiting agents can be polynucleotides, and in certain cases oligonucleotides or polypeptides, such as proteins or antibodies, for example.
The present invention also has for its object a process for the identification of an activated or inhibited agent by a polypeptide according to the invention, comprising:

a) the preparation of a recombinant vector comprising a polynucleotide according to the invention containing the coding SNP of the invention,
b) the preparation of host cells comprising a recombinant vector according to a),
c) putting host cells according to b) in the presence of an agent to be tested, and
d) the determination of the activating or inhibiting effect generated by the agent to be tested.

An agent activated or inhibited by the polypeptide of the invention is an agent that responds, respectively, to an activation or an inhibition in the presence of this polypeptide. The agents, activated or inhibited directly or indirectly by the polypeptide of the invention, can consist of polypeptides such as, for example, membranal or nuclear receptors, kinases and more preferably tyrosine kinases, transcription factor or polynucleotides.

Detection of diseases

The present invention also has for its object a process for the detection of the expression of a polypeptide according to the invention, comprising:

a) the detection of the presence or of the absence of a polynucleotide according to the invention, comprising the coding SNP of the invention, in the genome of the subject, and/or
b) the determination of the concentration of a polypeptide according to the invention in a subject.

The detection of a polynucleotide and/or the determination of the concentration of a polypeptide according to the invention can permit one to know if a subject is affected or at risk of being affected or, to the contrary, presents a partial resistance to the development of a disease, of an indisposition or of a disorder such as previously defined, relative to the expression of a polypeptide of the invention.
Preferably, in stage a) of the detection process mentioned above, the presence or absence of a polynucleotide containing the coding SNP such as previously defined is going to be detected.
The detection of the polynucleotide can be carried out starting from biological samples of the subject to be studied, such as cells, blood, urine, saliva, or starting from a biopsy or an autopsy of the subject to be studied. The genomic DNA can be used for the detection directly or after a PCR amplification, for example. RNA or cDNA can equally be used in a similar fashion.
It is then possible to compare the nucleotide sequence of a polynucleotide according to the invention with the nucleotide sequence detected in the genome of the subject.
The comparison of the nucleotide sequences can be carried out by sequencing, by DNA hybridization methods, by mobility difference of the DNA fragments on an electrophoresis gel with or without denaturing agents or by fusion temperature difference. See Myers et al., Science (1985) 230: 1242. Such modifications in the structure of the nucleotide sequence at a precise point can equally be revealed by nuclease protection tests, such as RNase and the S1 nuclease or also by chemical cleaving agents. See Cotton et al., Proc. Nat. Acad. Sci. USA (1985) 85:4397-4401. Oligonucleotide probes comprising a polynucleotide fragment of the invention can equally be used to conduct the screening.
Numerous methods well known to a person skilled in the art can be used to determine the expression of a polynucleotide of the invention and to identify the genetic variability of this polynucleotide. See Chee et al., Science (1996), Vol 274, pp 610-613.
The present invention equally has for its object the use of a polynucleotide according to the invention to carry out a genetic diagnosis of a disease or of a resistance to a disease linked to the presence, in one or several individuals of the human population, of the mutant allele encoded by a functional SNP according to the invention.
These diseases can be disorders and/or human diseases, such as disorder or diseases involving the immune system, inflammation and tumor cell invasion, psoriasis and rheumatoid arthritis, chronic polyarthritis, myelodysplastic syndrome, carcinoma such as hepatocellular carcinoma and osteosarcoma.
The determination of the polypeptide concentration according to the invention, in stage b), can equally help in the diagnosis of a disease, an indisposition or a disorder or, to the contrary, the resistance to a disease, an indisposition or a disorder in a subject by withdrawing a sample derived from that subject.
The increase or the decrease of the expression of the polypeptide can be measured by quantifying the level of RNA coding for this polypeptide, following methods well known to a person skilled in the art, for example, by PCR, RT-PCR, RNase protection, Northern blot, and other hybridization methods.
It is equally possible to determine the polypeptide concentration of the invention present in a biological sample of the subject by well known methods, for example, by radioimmunoassay, competitive binding tests, Western blot and ELISA tests.
Consecutively to stage b), the determined polypeptide concentration according to the invention can be compared with the natural protein concentration usually found in a subject.

A person skilled in the art can identify the threshold above or below which appears the sensitivity or, to the contrary, the resistance to the disease, the indisposition or the disorder evoked above, with the help of prior art publications or by conventional tests or assays, such as those that are previously mentioned.

Medications and treatments of diseases

The present invention also has for its object a medication containing, by way of active principle, a polypeptide according to the invention.

The invention also relates to the use of a polypeptide according to the invention, for the manufacture of a medication intended for the prevention or the treatment of different human disorders and/or diseases, such as disorder or diseases involving the immune system, inflammation and tumor cell invasion, psoriasis and rheumatoid arthritis, chronic polyarthritis, myelodysplastic syndrome, carcinoma such as hepatocellular carcinoma and osteosarcoma.

Certain of the compounds permitting to obtain the polypeptide according to the invention as well as the compounds obtained or identified starting from this polypeptide can likewise be used for the therapeutic treatment of the human body, i.e., by way of medication.

This is why the present invention also has for an object a medication containing, by way of active principle a polynucleotide according to the invention containing preferably the previously defined coding SNP, a previously defined recombinant vector, a previously defined host cell, a previously defined antibody and/or an activating and or inhibiting agent of a polypeptide according to the invention.

The invention also relates to the use of a polynucleotide according to the invention containing preferably the previously defined coding SNP, a previously defined recombinant vector, a previously defined host cell, a previously defined antibody and/or an activator and/or inhibitor agent of a polypeptide according to the invention, for the manufacture of a medication intended for the prevention or the treatment of different human disorders and/or diseases, such as disorders or diseases involving the immune system, inflammation and tumor cell invasion, psoriasis and rheumatoid arthritis, chronic polyarthritis, myelodysplastic syndrome, carcinoma such as hepatocellular carcinoma and osteosarcoma.

The dosage of a polypeptide and of the other compounds of the invention, useful as active principle, depends on the choice of the compound, the therapeutic indication, the mode of administration, the nature of the formulation, the nature of the subject and the judgment of the doctor.

When it is used as active principle, a polypeptide according to the invention is generally administered at doses ranging between 1 and 100 μg/kg of the subject, per day and according to the mode of administration.

The invention also has as an object a pharmaceutical composition that contains at least one above-mentioned compound, such as a polypeptide according to the invention, a polynucleotide according to the invention containing preferably the previously defined coding SNP, a previously defined recombinant vector, a previously defined host cell, a previously defined antibody and/or an activating and/or inhibiting agent of a polypeptide according to the invention, by way of active principle, as well as a pharmaceutically acceptable excipient.

In these pharmaceutical compositions, the active principle is advantageously present at physiologically effective doses.

These pharmaceutical compositions can be, for example, solids or liquids and be present in pharmaceutical forms currently used in human medicine, such as for example simple or coated tablets, gelcaps, granules, caramels, suppositories and preferably injectable preparations and powders for injectables. These pharmaceutical forms can be prepared according to typical methods.

The active agent(s) can be incorporated into typically employed excipients in these pharmaceutical compositions, such as talc, Arabic gum, lactose, starch, dextrose, glycerol, ethanol, magnesium stearate, cocoa butter, aqueous or non-aqueous vehicles, fatty substances of animal or vegetable origin, paraffinic derivatives, glycols, various wetting agents, dispersants or emulsifiers, preservatives.

The active agent(s) according to the invention can be employed alone or in combination with other compounds, such as therapeutic compounds such as IFNsα, cytokines such as interleukin, IL-8, for example.

The different formulations of the pharmaceutical compositions are adapted according to the mode of administration.

The pharmaceutical compositions can be administered by different routes of administration known to a person skilled in the art.

The invention equally has for an object a diagnostic composition that contains at least one above-mentioned compound, such as a polypeptide according to the invention, all or part of a polynucleotide according to the invention containing preferably the previously defined coding SNP, a previously defined recombinant vector, a previously defined host cell and/or a previously defined antibody.

This diagnostic composition may contain, for example, an appropriate excipient like those generally used in the diagnostic composition such as buffers and preservatives.

The present invention equally has as an object the use:

a) of a therapeutically effective quantity of a polypeptide according to the invention, and/or
b) of a polynucleotide according to the invention, and/or
c) of a host cell of the subject to be treated, previously defined, to prepare a medication intended to increase the expression or the activity, in a subject, of a polypeptide according to the invention.

Thus, to treat a subject who needs an increase in the expression or in the activity of a polypeptide of the invention, several methods are possible.

It is possible to administer to the subject a therapeutically effective quantity of a polypeptide of the invention and/or of the activating agent and/or activated such as previously defined, possibly in combination, with a pharmaceutically acceptable excipient.

It is likewise possible to increase the endogenous production of a polypeptide of the invention by administration to the subject of a polynucleotide according to the invention. For example, this polynucleotide can be inserted in a retroviral expression vector. Such a vector can be isolated starting from cells having been infected by a retroviral plasmid vector containing RNA encoding for the polypeptide of the invention, in such a fashion that the transduced cells produce infectious viral particles containing the gene of interest. See Gene Therapy and other Molecular Genetic-based Therapeutic Approaches, Chapter 20, in Human Molecular Genetics, Strachan and Read, BIOS Scientifics Publishers Ltd (1996).

In accordance with the invention, a polynucleotide containing the coding SNP such as previously defined is going to be preferably used.

It is equally possible to administer to the subject host cells belonging to him, these host cells having been taken and modified, preliminarily, so as to express the polypeptide of the invention, as previously described.

The present invention equally relates to the use:

a) of a therapeutically effective quantity of a previously defined immunospecific antibody, and/or
b) of a polynucleotide permitting inhibition of the expression of a polynucleotide according to the invention,

In order to prepare a medication intended to reduce the expression or the activity, in a subject, of a polypeptide according to the invention.

Thus, it is possible to administer to the subject a therapeutically effective quantity of an inhibiting agent and/or of an antibody such as previously defined, possibly in combination, with a pharmaceutically acceptable excipient.

It is equally possible to reduce the endogenous production of a polypeptide of the invention by administration to the subject of a complementary polynucleotide according to the invention permitting inhibition of the expression of a polynucleotide of the invention.

Preferably, a complementary polynucleotide containing the coding SNP such as previously defined can be used.

The present invention concerns also the use of a GCP-2 protein for the preparation of a medication for the prevention or the treatment of a patient having a disorder or a disease caused by a GCP-2 variant linked to the presence in the genome of said patient of a nucleotide sequence having at least 95% identity (preferably, 97% identity, more preferably 99% identity and particularly 100% identity) with the nucleotide sequence SEQ ID N° 1, provided that said nucleotide sequence comprises at least one of the following SNPs : g563t, c574g, c646g, g742t, a1026t, g1069c and preferably 1254-1255 Ins (t).

Preferably, said medication is used for the prevention or the treatment of one of the diseases selected from the group consisting of the disorder or diseases involving the immune system, inflammation and tumor cell invasion, psoriasis and rheumatoid arthritis, chronic polyarthritis, myelodysplastic syndrome, carcinoma such as hepatocellular carcinoma and osteosarcoma.

## EXPERIMENTAL PART

### Example 1 : Genotyping of the SNPs 1254-1255 Ins (t) in a population of individuals

The genotyping of SNPs is based on the principle of the minisequencing wherein the product is detected by a reading of polarized fluorescence. The technique consists of a fluorescent minisequencing (Technology FP-TDI or Fluorescence Polarization Template-direct Dye-terminator Incorporation).

The minisequencing consists of lengthening an oligonucleotide primer, placed just upstream of the site of the SNP, by dideoxynucleotides fluorolabeled using a polymerase enzyme. The resulting product of this lengthening is directly analyzed by a reading of polarized fluorescence.

The genotyping requires 5 stages:

1) Amplification by PCR
2) Purification of the PCR product by enzymatic digestion
3) Elongation of the oligonucleotide primer
4) Reading
5) Interpretation of the reading

1) The PCR amplification of the nucleotide sequence of the GCP-2 gene is carried out starting from genomic DNA coming from 268 of individuals of ethnically diverse origins.

These genomic DNAs were provided by the Coriell Institute in the United States.

The 268 individuals are distributed as follows:

| PHYLOGENIC POPULATION | SPECIFIC ETHNIC POPULATION | NUMBER OF INDIVIDUALS |
|---|---|---|
| African American | African American | 50 |
| Amerind | Southwestern American Indian | 5 |
| Amerdind | South American (Andes) | 10 |
| Carribean | Caribbean | 10 |
| European Caucasoid | North American Caucasian | 79 |
| European Caucasoid | Iberian | 10 |
| European Caucasoid | Italian | 10 |
| Mexican | Mexican | 10 |
| Northeast Asian | Chinese | 10 |
| Northeast Asian | Japanese | 10 |
| Non-European Caucasoid | Greek | 8 |
| Non-European Caucasoid | Indo-Pakistani | 9 |
| Non-European Caucasoid | Middle-Eastern | 20 |
| Southeast Asian | Pacific Islander | 7 |
| Southeast Asian | South Asian | 10 |
| South American | South American | 10 |

The genomic DNA coming from each one of these individuals constitutes a sample.

The PCR amplification is carried out starting from the following primers: SEQ ID N° 4 and SEQ ID N° 5.

The total reactional volume made use of for the PCR amplification is 5 µl per sample.

This reactional volume is composed of the reagents indicated in the following table:

| Supplier | Reference | Reactant | Initial Conc. | Vol. per tube (µl) | Final Conc. |
|---|---|---|---|---|---|
| Life Technology | Delivered with Taq | Buffer (X) | 10 | 0.5 | 1 |
| Life Technology | Delivered with Taq | $MgSO_4$ (mM) | 50 | 0.2 | 2 |
| AP Biotech | 27-2035-03 | dNTPs (mM) | 10 | 0.1 | 0.2 |
| | On request | Primer F ( µM) SEQ ID N° 3 | 10 | 0.1 | 0.2 |

(continued)

| Supplier | Reference | Reactant | Initial Conc. | Vol. per tube (µl) | Final Conc. |
|---|---|---|---|---|---|
| | On request | Primer R (µM) SEQ ID N° 4 | 10 | 0.1 | 0.2 |
| Life Technology | 11304-029 | Taq platinum | 5U/ µl | 0.02 | 0.1 U/reaction |
| | | $H_2O$ | Qsp 5 µl | 1.98 | |
| | | DNA (sample) | 2.5 ng/ µl | 2 | 5 ng/reaction |
| | | Total volume | | 5 µl | |

These reagents are distributed in a black PCR plate having 384 wells provided by ABGene (ref :TF-0384-k). The plate is sealed, centrifuged, then placed in a thermocycler for 384 well plates (Tetrad of MJ Research) and undergoes the following incubation: PCR Cycles: 1 min at 94° C, followed by 36 cycles composed of 3 stages (15 sec. at 94° C, 30 sec. at 56° C, 1 min at 68° C).

2) The PCR amplified product is then purified with the assistance of two enzymes: Shrimp Alkaline Phosphatase (SAP) and exonuclease I (Exo I). The first of these enzymes permits the dephosphorylation of the dNTPs which have not been incorporated during the PCR amplification, whereas the second eliminates the single stranded DNA residues, in particular the primers which have not been used during the PCR.

This digestion is done by addition, in each well of the PCR plate, of a reactional mixture of 5 µl per sample.

This reactional mixture is composed of the following reagents:

| Supplier | Reference | Reactant | Initial Conc | Vol. per tube (µl) | Final conc. |
|---|---|---|---|---|---|
| AP Biotech | E70092X | SAP | 1 U/ µl | 0.5 | 0.5/reaction |
| AP Biotech | 070073Z | Exo I | 10 U/ µl | 0.1 | 1/reaction |
| AP Biotech | Supplied with SAP | Buffer SAP (X) | 10 | 0.5 | 1 |
| | | $H_2O$ | Qsp 5 µl | 3.9 | |
| | | PCR amplifiant | | 5 µl | |
| | | Vol total | | 10 µl | |

Once filled, the plate is sealed, centrifuged, then placed in a thermocycler for 384 well plates (Tetrad of MJ Research) and undergoes the following incubation: Digestion SAP-EXO : 45 min at 37° C, 15 min at 80° C.

The elongation or minisequencing stage is then carried out on this product of PCR digested by addition of a reactional mixture of 5 µl per prepared sample.

The elongation or minisequencing stage is then carried out as indicated in the following table:

| Supplier | Reference | Reactant | Initial conc. | Vol. per tube (µl) | Final conc. |
|---|---|---|---|---|---|
| Clean preparation | | Elongation Buffer[1] (X) | 5 | 1 | 1 |
| Life Technologies | On request | Miniseq Primer (µM) A or B | 10 | 0.5 | 1 |

[1] The elongation buffer: The elongation buffer 5X is composed of Tris-HCl pH 9 at 250 mM, of KCl at 250 mM, of NaCl at 25 mM, of $MgCl_2$ at 10 mM and of glycerol at 40 %.

(continued)

| Supplier | Reference | Reactant | Initial conc. | Vol. per tube (µl) | Final conc. |
|---|---|---|---|---|---|
| AP Biotech | 27-2051 (61,71,81)-01 | ddNTPs[2] (µM) 2 non labeled | 2.5 of each | 0.25 | 0.125 of each |
| NEN | Nel 472/5 and Nel 492/5 | ddNTPs[2] (µM) 2 labeled Tamra and R110 | 2.5 of each | 0.25 | 0.125 of each |
| AP Biotech | E79000Z | Thermo-sequenase | 3.2 U/µl | 0.125 0.125 | 0.4 U/reaction |
| | | H2O | Qsp 5 µl | 3.125 | |
| | | PCR digested | | 10 µl | |
| | | Vol total | | 15 µl | |

[2] ddNTPs : For the ddNTPs, a mixture of the 4 bases is carried out according to the polymorphism studied. Only the 2 bases of interest (G/T) composing the functional SNP are labeled, either in Tamra, or in R110. The mixture of ddNTPs is composed of:

- 2.5 µM of ddCTP non labeled,
- 2.5 µM of ddATP non-labeled,
- 2.5 µM of ddTTP (1.875 µM of ddTTP non labeled and 0.625 µM of ddTTP Tamra labeled),
- 2.5 µM of ddGTP (1.875 µM of ddGTP non labeled and 0.625 µM of ddGTP R110 labeled),

Once filled, the plate is sealed, centrifuged, then placed in a thermocycler for 384 well plates (Tetrad of MJ Research) and undergoes the following incubation: Elongation cycles : 1 min. at 93° C, followed by 35 cycles composed of 2 stages (10 sec. at 93° C, 30 sec. at 55° C).

After the last stage in the thermocycler, the plate is directly placed on a polarized fluorescence reader of type Analyst® HT of LJL Biosystems Inc. The plate is read using Criterion Host® software by using two methods. The first permits reading the Tamra labeled base by using emission and excitation filters specific for this fluorophore (excitation 550-10 nm, emission 580-10 nm) and the second permits reading the R110 labeled base by using the excitation and emission filters specific for this fluorophore (excitation 490-10 nm, emission 520-10 nm). In the two cases, a dichroic double mirror (R110/Tamra) is used and the other reading parameters are :

Z-height: 1.5 mm
Attenuator: out
Integration time: 100,000 µsec.
Raw data units: counts/sec
Switch polarization: by well
Plate settling time: 0 msec
PMT setup: Smart Read (+), sensitivity 2
Dynamic polarizer: emission
Static polarizer: S

A file result is thus obtained containing the calculated values of mP (milliPolarization) for the Tamra filter and that for the R110 filter. These mP values are calculated starting from intensity values obtained on the parallel plane (//) and on the perpendicular plane (⊥) according to the following formula :

$$MP = 1000(// - g\perp)/(// + g\perp).$$

In this calculation, the value ⊥ is weighted by a factor g. It is a machine parameter that must be determined experimentally beforehand.

4.1) and 5.1) Interpretation of the reading and determination of the genotypes.

The mP values are reported on a graph using Microsoft Inc. Excel software, and/or Allele Caller® software developed by LJL Biosystems Inc.

On the abscissa is indicated the mP value of the Tamra labeled base, on the ordinate is indicated the mP value of the R110 labeled base. A strong mP value indicates that the base labeled with this fluorophore is incorporated and, conversely, a weak mP value reveals the absence of incorporation of this base.

Up to three homogenous groups of nucleotide sequences having different genotypes, as indicated in Figure 1 are obtained.

The use of the Allele Caller® software permits, once the identification of the different groups is carried out, to directly extract the genotype defined for each individual in table form.

The sequences of the two minisequencing primers necessary for the genotyping were determined in a way to be placed upstream of the site of a SNP according to the invention. The PCR product that contains the SNP being a double stranded DNA product, the genotyping can therefore be done either on the sense strand or on the antisense strand. The selected primers are manufactured by Life Technologies Inc.

The minisequencing primers are the following:

Sense primer: (A): CAGTGCTCCAAGGTGGAAGT
Antisense primer: (B): AGCAACACAGGAGAACTTACACCA

The minisequencing of the SNP 1254-1255 Ins (t) was first validated over 48 samples, then genotyped over the set of the population of individuals composed of 268 individuals and 11 negative controls.

Several conditions for the minisequencing have been tested and the following condition was retained for the genotyping : Sense primer + ddGTP-R110 + ddTTP-Tamra.

After the complete carrying out of the genotyping process, the determination of the genotypes of the individuals of the population of individuals for the functional SNP studied here was carried out using the graph represented in Figure 1

This genotype is in theory either homozygote GG, or heterozygote GT, or homozygote TT in the tested individuals. In reality, and as shown below, the homozygote genotype TT is not detected in the population of individuals.

The results of the controls, of the distribution of the determined genotypes in the population of individuals and the calculation of the different allelic frequencies for this functional SNP are presented in the following tables:

| **Number of individuals** | | **Number of controls** | | **Percentage of success** |
|---|---|---|---|---|
| tested | genotyped | tested | Validated | |
| 268 | 266 | 11 | 8 | 98.2% |

| POPULATION | | | Allelic frequency | | | Genotype GG | | Genotype TG | | Genotype TT | |
|---|---|---|---|---|---|---|---|---|---|---|---|
| | N | % | % | 95% CI | | N | % | N | % | N | % |
| African American | 50 | 18.7 | 0.0 | - | - | 50 | 100 | 0 | 0.0 | 0 | 0.0 |
| Amerind | 15 | 5.6 | 0.0 | - | - | 15 | 100 | 0 | 0.0 | 0 | 0.0 |
| Caribbean | 10 | 3.7 | 0.0 | - | - | 10 | 100 | 0 | 0.0 | 0 | 0.0 |
| European Caucasoid | 99 | 36.9 | 0.5 | 0 | 1.5 | 96 | 99.0 | 1 | 1.0 | 0 | 0.0 |
| Mexican | 10 | 3.7 | 0.0 | - | - | 10 | 100 | 0 | 0.0 | 0 | 0.0 |
| Non-European Caucasoid | 37 | 13.8 | 2.7 | 0 | 6.4 | 35 | 94.6 | 2 | 5.4 | 0 | 0.0 |
| Northeast Asian | 20 | 7.5 | 0.0 | - | - | 20 | 100 | 0 | 0.0 | 0 | 0.0 |
| South American | 10 | 3.7 | 0.0 | - | - | 10 | 100 | 0 | 0.0 | 0 | 0.0 |
| Southeast Asian | 17 | 6.3 | 0.0 | - | - | 17 | 100 | 0 | 0.0 | 0 | 0.0 |
| Total | 268 | 100 | 0.6 | 0 | 1.2 | 263 | 98.9 | 3 | 1.1 | 0 | 0.0 |

In the above table,

- N represents the number of individuals,
- % represents the percentage of individuals in the specific sub-population,
- the allelic frequency represents the percentage of the mutated allele in the specific sub-population,
- 95 % IC represents the minimal and maximal interval of confidence at 95 %.

By examining these results by population, it is observed that the three heterozygote individuals GT are from the sub-population European Caucasoid and Non-European Caucasoid of the population of individuals.

## Claims

**1.** Isolated polynucleotide comprising:

a) a nucleotide sequence having at least 80 % identity with the sequence SEQ ID N° 1 or its coding sequence, it being understood that this nucleotide sequence contains the SNP : 1254-1255 Ins (t); or
b) a nucleotide sequence complementary to a nucleotide sequence under a).

**2.** Isolated polynucleotide comprising:

a) the nucleotide sequence SEQ ID N° 1 or its coding sequence, it being understood that each one of these sequences comprises the SNP : 1254-1255 Ins (t); or
b) a nucleotide sequence complementary to a nucleotide sequence under a).

**3.** Polynucleotide according to any one of claims 1 to 2, **characterized in that** it consists of the sequence SEQ ID N° 1 or its coding sequence, it being understood that each one of the sequences contains the SNP 1254-1255 Ins (t).

**4.** Isolated polynucleotide comprising:

a) a nucleotide sequence having at least 80 % identity with the nucleotide sequence SEQ ID N°1, it being understood that this sequence contains at least one of the following SNPs : g563t, c574g, c646g, g742t, a1026t, g1069c, or
b) a nucleotide sequence complementary to the nucleotide sequence under a).

**5.** Isolated polynucleotide consisting of a part of a polynucleotide according to any one of claims 1 to 4, it being understood that the sequence of said isolated polynucleotide contains at least one of the following SNPs : g563t, c574g, c646g, g742t, a1026t, g1069c, 1254-1255 Ins (t), said isolated nucleotide being composed of at least 10 nucleotides.

**6.** Isolated polynucleotide, **characterized in that** it codes for a polypeptide comprising:

a) the amino acid sequence SEQ ID N° 3,
b) the amino acid sequence comprising the amino acids included between positions 38 and 115 of the amino acid sequence SEQ ID N° 3, or
c) the amino acid sequence comprising the amino acids included between positions 46 and 115 of the amino acid sequence SEQ ID N° 3.

**7.** Use of all or part of a polynucleotide according to any one of claims 1 to 6, to identify, hybridize and/or amplify all or part of a polynucleotide consisting of:

- the sequence SEQ ID N° 1, it being understood that this sequence contains at least one of the following SNPs : g563t, c574g, c646g, g742t, a1026t, g1069c, 1254-1255 Ins (t), and/or
- the coding sequence of the nucleotide sequence SEQ ID N° 1, it being understood that this sequence contains the following SNP : 1254-1255 Ins (t).

**8.** Use of all or part of a polynucleotide according to any one of claims 1 to 6, for the genotyping of a nucleotide sequence which has 90 to 100 % identity with the nucleotide sequence SEQ ID N°1 and which comprises at least one of the following SNPs : g563t, c574g, c646g, g742t, a1026t, g1069c, 1254-1255 Ins (t).

**9.** Use according to claim 8, in which the genotyping is carried out by minisequencing.

**10.** Recombinant vector comprising a polynucleotide according to one of claims 1 to 6.

**11.** Host cell comprising a recombinant vector according to claim 10.

**12.** Process for the preparation of a polypeptide, **characterized in that** a host cell according to claim 11 is cultivated in a culture medium and said polypeptide is isolated from the culture medium.

**13.** Isolated polypeptide comprising an amino acid sequence having at least 80 % identity with:

a) the amino acid sequence SEQ ID N° 3,
b) the amino acid sequence containing the amino acids included between positions 38 and 115 of the amino acid sequence SEQ ID N° 3,
c) the amino acid sequence containing the amino acids included between positions 46 and 115 of the amino acid sequence SEQ ID N° 3.

**14.** Polypeptide according to claim 13, **characterized in that** it comprises:

a) the amino acid sequence SEQ ID N° 3,
b) the amino acid sequence containing the amino acids included between positions 38 and 115 of the amino acid sequence SEQ ID N° 3, or
c) the amino acid sequence containing the amino acids included between positions 46 and 115 of the amino acid sequence SEQ ID N° 3.

**15.** Polypeptide according to any one of claims 13 and 14, **characterized in that** it consists of:

a) the amino acid sequence SEQ ID N° 3,
b) the amino acid sequence containing the amino acids included between positions 38 and 115 of the amino acid sequence SEQ ID N° 3, or
c) the amino acid sequence containing the amino acids included between positions 46 and 115 of the amino acid sequence SEQ ID N° 3.

**16.** Process for obtaining an immunospecific antibody, **characterized in that** it is obtained by immunization of an animal with a polypeptide according to any one of claims 13 to 15.

**17.** Immunospecific antibody for a polypeptide according to any one of claims 13 to 15.

**18.** Process for the detection of the expression and/or of the activity of a polypeptide according to any one of claims 13 to 15, comprising :

a) the detection of the presence or of the absence of a polynucleotide according to any one of claims 1 to 3 and 6 in the genome of the subject, and/or
b) the determination of the concentration of a polypeptide according to any one of claims 13 to 15, in a biological sample of the subject.

**19.** Medication comprising by way of active agent at least one polypeptide according to any one of claims 13 to 15.

**20.** Use of a polypeptide according to any one of claims 13 to 15, for the preparation of a medication intended for the prevention or the treatment of one of the diseases selected from the group consisting of the disorders or diseases involving the immune system, inflammation and tumor cell invasion, psoriasis and rheumatoid arthritis, chronic polyarthritis, myelodysplastic syndrome, carcinoma such as hepatocellular carcinoma and osteosarcoma.

**21.** Medication comprising by way of active agent at least one polynucleotide according to any one of claims 1 to 6, a recombinant vector according to claim 10, a host cell according to claim 11 and/or an antibody according to claim 17.

**22.** Use of a polynucleotide according to any one of claims 1 to 6, a recombinant vector according to claim 10, a host cell according to claim 11 and/or an antibody according to claim 17, for the preparation of a medication intended for the prevention or the treatment of one of the diseases selected from the group consisting of the disorders or diseases involving the immune system, inflammation and tumor cell invasion, psoriasis and rheumatoid arthritis, chronic polyarthritis, myelodysplastic syndrome, carcinoma such as hepatocellular carcinoma and osteosarcoma.

**23.** Pharmaceutical composition containing by way of active agent at least one polypeptide according to any one of claims 13 to 15, all or part of a polynucleotide according to any one of claims 1 to 6, a recombinant vector according to claim 10, a host cell according to claim 11 and/or an antibody according to claim 17, as well as a pharmaceutically acceptable excipient.

**24.** Diagnostic composition containing by way of active agent at least one polypeptide according to any one of claims 13 to 15, all or part of a polynucleotide according to any one of claims 1 to 6, a recombinant vector according to claim 10, a host cell according to claim 11 and/or an antibody according to claim 17.

**25.** Use of a GCP-2 protein for the preparation of a medication for the prevention or the treatment of a patient having a disorder or a disease caused by a GCP-2 variant linked to the presence in the genome of said patient of a nucleotide sequence having at least 95% identity with the nucleotide sequence SEQ ID N° 1, provided that said nucleotide sequence comprises at least one of the following SNPs : g563t, c574g, c646g, g742t, a1026t, g1069c, 1254-1255 Ins (t).

Figure 1

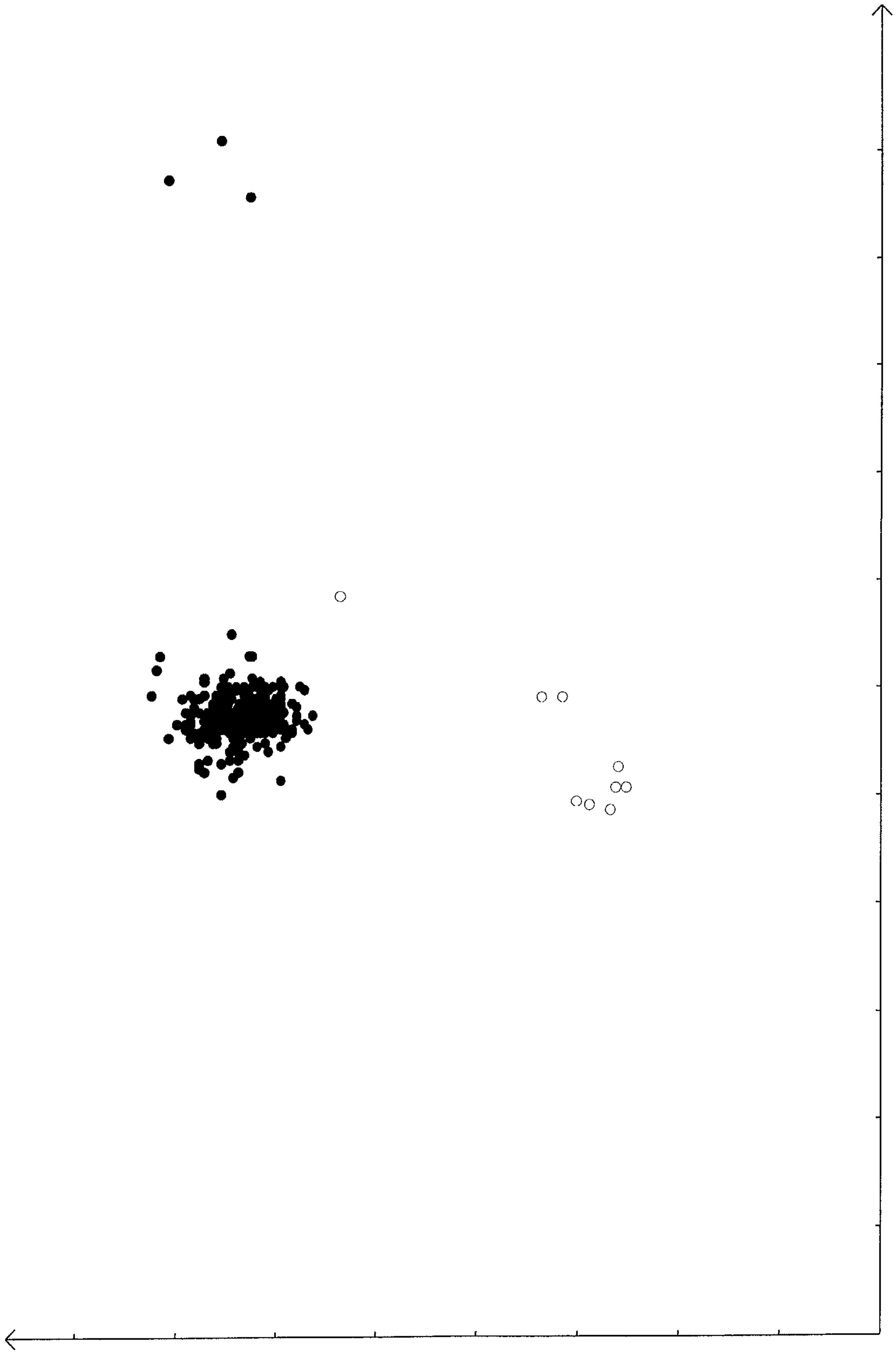

European Patent Office

# EUROPEAN SEARCH REPORT

Application Number
EP 01 40 2950

| Category | DOCUMENTS CONSIDERED TO BE RELEVANT<br>Citation of document with indication, where appropriate, of relevant passages | Relevant to claim | CLASSIFICATION OF THE APPLICATION (Int.Cl.7) |
|---|---|---|---|
| X | WO 98 11227 A (INCYTE PHARMA INC ;COLEMAN ROGER (US); BANDMAN OLGA (US); MURRY LY) 19 March 1998 (1998-03-19)<br>see whole doc. esp. claims<br>--- | 1-25 | C07K14/52<br>C12Q1/68 |
| X | PROOST P ET AL: "HUMAN AND BOVINE GRANULOCYTE CHEMOTACTIC PROTEIN-2: COMPLETE AMINO ACID SEQUENCE AND FUNCTIONAL CHARACTERIZATION AS CHEMOKINES"<br>BIOCHEMISTRY, AMERICAN CHEMICAL SOCIETY. EASTON, PA, US,<br>vol. 32, 1993, pages 10170-10177, XP002049871<br>ISSN: 0006-2960<br>--- | 1-5 | |
| X | US 5 840 524 A (PROOST PAUL ET AL) 24 November 1998 (1998-11-24)<br>see whole doc. esp. claims<br>--- | 1-25 | |
| X | DATABASE SWISS-PROT 'Online!<br>U81234, 6 January 1999 (1999-01-06)<br>NI J. : "cloning,sequencing and biological chracterization of a C-X-C chemokine, CKA-3"<br>XP002196466<br>* abstract *<br>--- | 1-25 | TECHNICAL FIELDS SEARCHED (Int.Cl.7)<br>C07K<br>C12Q |
| A | ROVAI L E ET AL: "CLONING AND CHARACTERIZATION OF THE HUMAN GRANULOCYTE CHEMOTACTIC PROTEIN-2 GENE"<br>JOURNAL OF IMMUNOLOGY, THE WILLIAMS AND WILKINS CO. BALTIMORE, US,<br>vol. 158, June 1997 (1997-06), pages 5257-5266, XP002906957<br>ISSN: 0022-1767<br>* the whole document *<br>---<br>-/-- | | |

The present search report has been drawn up for all claims

| Place of search | Date of completion of the search | Examiner |
|---|---|---|
| MUNICH | 17 April 2002 | Mueller, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

European Patent Office

# EUROPEAN SEARCH REPORT

Application Number
EP 01 40 2950

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| A | WUYTS A. ET AL.,: "purification and identification of human and mouse granulocyte chemotactic protein 2-isoforms" METHODS IN EZYMOLOGY, vol. 287, 1997, pages 12-33, XP001068383 see whole doc. esp. page 19 ff. ----- | | |
| | | | TECHNICAL FIELDS SEARCHED (Int.Cl.7) |

The present search report has been drawn up for all claims

| Place of search | Date of completion of the search | Examiner |
|---|---|---|
| MUNICH | 17 April 2002 | Mueller, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT ON EUROPEAN PATENT APPLICATION NO. EP 01 40 2950

This annex lists the patent family members relating to the patent documents cited in the above-mentioned European search report.
The members are as contained in the European Patent Office EDP file on
The European Patent Office is in no way liable for these particulars which are merely given for the purpose of information.

17-04-2002

| Patent document cited in search report | Publication date | Patent family member(s) | Publication date |
|---|---|---|---|
| WO 9811227 A | 19-03-1998 | AU 4266197 A | 02-04-1998 |
| | | EP 0958365 A1 | 24-11-1999 |
| | | JP 2001500019 T | 09-01-2001 |
| | | WO 9811227 A1 | 19-03-1998 |
| US 5840524 A | 24-11-1998 | AU 5628394 A | 22-06-1994 |
| | | EP 0804486 A2 | 05-11-1997 |
| | | WO 9412537 A2 | 09-06-1994 |

EPO FORM P0459

For more details about this annex : see Official Journal of the European Patent Office, No. 12/82